# EUROPEAN PATENT APPLICATION

(11) **EP 4 804 203 A1**
(43) Date of publication of application: **09.09.2026**
(21) Application number: 25161195.0
(22) Date of filing: 03.03.2025
(51) Int. Cl.: G16H 40/63, A61B 5/00, F16M 11/18

(54) **DEVICE, SYSTEM, METHOD AND COMPUTER PROGRAM FOR AUTOMATICALLY ADJUSTING THE POSITION AND/OR ORIENTATION OF A MONITOR**

(71) Applicant: Koninklijke Philips N.V., 5656 AE Eindhoven (NL)
(72) Inventor: FRIEDRICH, Dirk, 5656 AG Eindhoven (NL); SPEICH, Alexandra, 5656 AG Eindhoven (NL); GALL, Tobias, 5656 AG Eindhoven (NL); VAN LIESHOUT, Ron Martinus Laurentius, 5656 AG Eindhoven (NL)
(74) Representative: Philips Intellectual Property & Standards

(57) **Abstract**

The present invention relates to device (120), system and method for automatically adjusting the position and/or orientation of a monitor. The device (120) comprises a holder (122) configured to hold a monitor (110) configured to display subject information related to a subject's physiological condition, wherein the holder (122) is configured to be in a parking state and a use state; a drive mechanism (124) configured to move the holder (122) from the parking state to the use state; and a control unit (126) configured to obtain subject information (114) indicating whether the subject (200) is in a critical state and to control the drive mechanism to move the holder (122) from the parking state to the use state if the subject information (114) indicates that the subject (200) is in a critical state.

## Description

### FIELD OF THE INVENTION

The present invention relates to a device, a system, a method and a computer program for automatically adjusting the position and/or orientation of a monitor that is configured to display subject information related to a subject's physiological condition.

### BACKGROUND OF THE INVENTION

Patient monitoring is used to monitor subjects' (e.g. patients') physiological condition (e.g. health state) to allow for clinical intervention if a critical state is detected. For example, subject information related to a subject's physiological condition, e.g. vital signs such as heart rate, blood oxygen saturation or respiration may be monitored. Based on such vital signs, clinical decisions can be made. For example, cardiac arrythmia, which causes the heart rhythm to be irregular, may result in a life-threatening situation for the patient. Monitoring the heart rate allows for prompt clinical interventions, e.g., by administering medication.

Nowadays, it is common to monitor patients not only in hospitals but also during transport, e.g., transport to the hospital or intrahospital transport. For example, a patient may be transported from the scene of an accident to the hospital. Also, a patient may be transported within the hospital to or from an operating room (also called intra-hospital transport). Interhospital transport is often conducted in order to take patients to specialized hospitals or health care centers, e.g., to a hospital specialized in cardiology. Patients may be transported, e.g., by ambulance, helicopter or plane.

Transport poses a particular risk to the patient as the likelihood of critical patient conditions is increased during transport. This may be due to the fact that the likelihood of health deterioration is higher, e.g., during or after posture changes of the patient and/or due to environmental changes. It has been also found that the risk for critical patient conditions during transport increases with the severity of the illness. Therefore, appropriate patient monitoring is especially important during transport of a patient to reduce the risk to the patient.

However, patient monitoring during transport may be cumbersome as space during patient transport, e.g., during inter-hospital transport or transport to a first hospital in an ambulance or helicopter, is usually limited. The same holds for intra-hospital transport where space on and near the bed of the patient may be limited. For example, patient monitoring equipment may block access to the patient during transport or may collide with the patient or caregiver. Working in little space in combination with possible time pressure present the caregiver with a challenge.

US 2007/0180129 A1 discloses a system and method for communicating critical and non-critical patient/medical information by visual display units such that the caregiver is able to freely move about the workplace and/or patient care area while maintaining visual contact with one or more of the visual display units.

US 2006/0071135 A1 discloses an apparatus and method for automatically positioning a device. A sensor detects the position of a user. In response to signals from the sensor, a processor determines an ideal position for use of the device.

US 2012/0013723 A1 discloses a support system and method for automatically positioning and aligning a display unit, preferably a monitor, depending on the position of a viewer.

### SUMMARY OF THE INVENTION

It is an object of the present invention to provide a device, a system, a method and a computer program allowing for user-friendly patient monitoring allowing to improve patient safety, in particular in confined spaces.

In a first aspect of the present invention a device for automatically adjusting the position and/or orientation of a monitor is presented, the device comprising:
- a holder configured to hold a monitor configured to display subject information related to a subject's physiological condition, wherein the holder is configured to be in a parking state and a use state;
- a drive mechanism configured to move the holder from the parking state to the use state; and
- a control unit configured to obtain subject information indicating whether the subject is in a critical state and to control the drive mechanism to move the holder from the parking state to the use state if the subject information indicates that the subject is in a critical state.

In a further aspect of the present invention a system for automatically adjusting position and/or orientation of a monitor is presented, the system comprising:
- a device for automatically adjusting the position and/or orientation of a monitor as disclosed herein; and
- a monitor configured to display subject information related to a subject's physiological condition, the monitor comprising a display configured to display the subject information.

In another aspect of the present invention, a method for automatically adjusting the position and/or orientation of a monitor is presented, the method comprising:
- obtaining subject information indicating whether the subject is in a critical state;
- controlling a driving unit to move a holder configured to hold a monitor configured to display subject information related to a subject's physiological condition from a parking state to a use state if the subject information indicates that the subject is in a critical state.

In yet a further aspect of the present invention, there is provided a corresponding computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

In intra-hospital transport the caregiver places the monitor at a specific place on the bed, sometimes without any support or with a support that is not accessible for all the caregivers (because it is static), preferably near the patient's head. This may cause a number of problems. A touch display could be unintentionally actuated by the patient or caregiver, which can result in changed/wrong settings, disabled alarms, changed mode of the device, etc. Further, the monitor may fall down, damaging the device or hurting the patient by pulling the cables or hurting the caregiver's foot, etc. The monitor may be covered by a blanket or other items. The loudspeaker for issuing alarms may be suppressed by a blanket and the display may not be in sight for the responsible caregiver. A static monitor-mount has limited flexibility. The present invention addresses and overcomes one or more (preferably all) of these problems.

The present invention is based on the findings that a caregiver needs to know the status of a subject during transport while the caregiver has limited time, space and/or access to the patient to adjust a monitor, such as a patient monitor or a display unit (e.g. external from or integrated into a patient monitor), during transport and/or a critical situation. The present invention is based on the idea of automatically adjusting the position and/or orientation of the monitor depending on subject information which indicates whether the subject is in a (clinical) critical state. The monitor may be adjusted by automatically controlling the holder configured to hold the monitor.

In the context of the present invention, the term "monitor" shall be understood broadly in the sense that it is able to at least display subject information related to a subject's physiological condition (e.g. vital signs, physiological information, sensor signals over time, health score, etc.; in general, all information that may be acquired and displayed by a patient monitor). The "monitor" may thus be a (simple) display unit that displays such subject information, e.g. as provided (transmitted wirelessly or via a wired connection) from a patient monitor or any other entity, or it may be a patient monitor, as e.g. provided in a hospital or an ambulance.

The subject information is obtained (i.e. retrieved or received) by the control unit, e.g., from a patient monitor and/or sensors or other equipment. Depending on whether the subject is in a critical state (critical condition) or not, the control unit automatically controls the drive mechanism such that the holder is in a parking state or a use state. If the subject information indicates that the state of the subject is non-critical (e.g., stable), the holder may be in the parking state. If the subject information indicates that the state of the subject is critical, the drive mechanism moves the holder from the parking state to the use state. If the subject information indicates that the state of the subject is non-critical again, e.g., due to clinical intervention, after a predefined time elapse and/or the user indicates that the state of the subject is non-critical, the drive mechanism may move the holder from the use state back to the parking state.

In an embodiment, a predictive algorithm may be used to indicate or predict a "pre-critical" state. Such a predictive algorithm generally has a probability and timing (actionability) aspect. By using these aspects the activation of the correct monitor position can be done during transport if the critical situation will happen during transport. In case a predictive algorithm is used, the output of this algorithm may be that a critical situation of the patient will happen within a certain time interval with a certain probability. Therefore, the device according to the present invention may switch from the parking state to the use state at a certain point in time in order to inform the caregiver and enable him/her to prepare for this situation, depending on which parameters will be critical and how actionable this situation is during transport. In preferred embodiment, the definition of this "pre-critical" state may be adjusted depending on the threshold for the probability and the time interval.

A critical state of the subject may be a state which requires attention and/or assessment of the state of the subject and/or clinical intervention by the user. This may in particular require providing the user with one or more parameters of the subject information on a display of the monitor. For example, the state of the subject may require that the user checks the state of the subject to determine whether clinical intervention is necessary. For example, the subject information may indicate that sensor information is lacking, e.g., because a sensor loosened and needs to be reattached or because additional measurement is advisable. The subject information may also indicate to the user that administration of medication is scheduled and/or that the state of the patient requires administration of (additional) medication. Furthermore, the critical state of the subject may indicate that the subject experiences a life-threatening situation. The user may be alerted to a critical state by an alarm, e.g., information displayed on the display of the patient monitor or by an audible signal.

The parking state may be a state in which the risk of damage of the holder and/or monitor (e.g., due to falling down, hitting anything in the surrounding during intra-hospital transport where the monitor may be held at the bed, etc.), unintended interaction with the monitor (e.g., by pressing buttons or by operating a touch screen), collision of the user, the subject (e.g., collision with the head) and/or equipment with the holder and/or monitor, and/or contamination of the monitor may be relatively little, in particular reduced compared to the use state. The parking state may be, for example, a state in which the holder is statically positioned and/or orientated or a state in which the holder and/or monitor are in the user's way as little as possible. For example, in the parking state, the holder and the monitor may be positioned close to an edge of a patient's bed and/or at relatively safe distance to the patient and/or caregiver. Optionally, the monitor may be positioned in a housing which may protect the monitor or anything in the surrounding from damage (e.g., by collision or by falling down), unintended operation and/or contamination. Another example of the parking state may be that a touch screen/display is placed close to and opposite a defined area of the patient bed, in order to avoid the above-mentioned problems.

The use state may be a state configured for interaction of the user with the monitor. In particular, the use state may allow the user to have a better view onto the monitor (in particular onto the display of the monitor) and/or to operate the monitor more conveniently than in the parking state. **In** other words, the use state may be a state where the user has, from a basic point of view, a correct view of most of and/or the important vital signs (i.e. monitoring). Furthermore, the use state may be a state that provides the ability to interact with the monitor (e.g. to set alarm limits, to acknowledge/silent an alert, change settings, retrieve other information for display, etc.).

The driving mechanism may be, e.g., an arm assembly comprising a plurality of joints, wherein the holder is fastened to the arm assembly. The arm assembly may allow moving and orientating the holder with all six degrees of freedom (three degrees of freedom of translation and three degrees of freedom of rotation). Alternatively, the arm assembly may allow for only, e.g., three degrees of freedom of translation and one degree of freedom of rotation, wherein the holder comprises a receptacle configured to receive the monitor and the receptacle is configured to orientate the monitor with the two remaining degrees of freedom of rotation. In an implementation, holders, arms, driving mechanisms, etc. as conventionally known may be used for this purpose.

The user may be a user of the monitor and/or a caregiver of the subject, such as an emergency doctor or paramedic.

Optionally, the control unit is configured to control the drive mechanism to set the holder from the use state to the parking state if no critical state of the subject is present and/or a critical state of the subject is no longer present. That a critical state is no longer present may be, e.g., determined by the control unit (e.g., by evaluating subject information comprising vital signs of the subject, for example by determining that a threshold is no longer violated, or due to a set time elapse, etc.) and/or set by the user (e.g., by pressing a button on the holder, operating the monitor (e.g., a touch screen), operating a remote control and/or voice control etc.).

According to the present invention, the control unit controls the holder to be in the parking state or in the use state based on the subject information, therefore allowing for a flexible positioning of the monitor. The monitor may be only in the use state if necessary and/or only as long as necessary. In the case that no attention from the caregiver is necessary, the holder may be in the parking state. As long as the holder is in parking state, the user may rely on that the subject is likely in a non-critical condition. This may indicate to the user that less high attention is required such that the user does not have to be constantly at maximum concentration. This may allow the user to better manage time periods with very high concentration and time periods with less high concentration. Furthermore, less distraction of the user may be ensured such that the user may focus on the subject. Also, the risk of collisions with the holder and/or monitor and/or damage of the monitor may be reduced, e.g., while the user provides routine clinical care during transport.

In an embodiment, the subject information comprises one or more of vital sign information of the subject, an alert, a trend and/or a predictive parameter related to vital sign information. Based on a predictive algorithm and/or an early warning score (EWS), the holder and/or the monitor may be in the parking state during "safe time periods". The vital sign information may be obtained by a sensor deployed on the subject. With respect to the EWS, an actionability/escalation pathway , particularly during transport, may be taken into account.

In the context of the present invention as disclosed therein, the term "vital sign information of the subject" refers to information indicating the status of the subject's vital functions and thus on the subject's state of health. The vital sign information can be a time-dependent signal used to monitor the subject over time. For example, the vital sign information is one or more of a pulse rate, body temperature, respiration rate, SpO2, and blood pressure (or information related to any other vital sign).

In an embodiment, the control unit is configured to control the drive mechanism to automatically adjust position and/or an orientation of the holder in the use state. By automatically, in particular dynamically, adjusting the position and/or orientation of the holder, the position and/or orientation of the monitor may be adjusted as well. In particular, the position and/or orientation of the holder may be adjusted depending on the needs of the user and/or the subject.

In another embodiment, the device further comprises a recognition unit configured to identify a user, in particular a caregiver of the subject, wherein the control unit is configured to control the drive mechanism to automatically adjust position and/or an orientation of the holder based on position and/or pose and/or movement of the user. Therefore, the user may move relatively freely without having to (manually) adjust the holder. The user may maintain view onto the monitor (in particular the display of the monitor) without interrupting the workflow. Thus, it may be ensured that the user is permanently provided with the relevant information, when the holder is in the use state, even if the user changes position and/or pose and/or moves. For example, the drive mechanism may move the holder to maintain a set distance to the user as long as possible (while at the same time avoiding collisions with a wall, other objects and/or the subject) and/or the drive mechanism may automatically move the holder to clear space if the user, e.g., stretches out his arm in order to administer medication to the subject or if the user leans over the patient.

In a further embodiment, the recognition unit is configured to identify the user by face recognition, eye movement tracking and/or real time locating, in particular a radio frequency identification, RFID, tag, a near field communication, NFC, tag and/or an optically readable information tag.

For example, face recognition software may be trained in advance to recognize the user, e.g., the caregiver who will take care of the patient during transport. Also, an eye gazing pattern may be used to distinguish the user from non-users. The recognition unit may be configured to compare an eye gazing pattern expected for a user familiar using the device and/or medical equipment with the eye gazing pattern of a potential user and/or an eye gazing pattern expected when presented with the medical condition (for example, when the patient has a certain criticality it is expected that the eye gazing pattern will move between and/or select different vital signs). This may allow to distinguish, e.g., the subject and/or a potential user (e.g., a secondary caregiver) from the user (e.g., primary caregiver). For example, the user may be identified by an eye gazing pattern related to one or more parameters of the subject information displayed on the display of the monitor or an eye gazing pattern related to an alert and/or a notification on the display of the monitor. The optical readable information tag may be, for example an identity card of the user, a barcode, a QR-code and/or markers attached to the user (e.g., pinned to clothing of the user, plaster-like detachably glued to the skin and/or to a headgear). The RFID tag, the NFC tag and/or the optical readable information tag may be held to a defined position (e.g., near the monitor) to check in a desired user.

Alternatively or additionally, one of many possible users may be chosen on a display (e.g., the display of the monitor) which shows potential users, e.g., identified by all identity cards being in range. If the subject is accompanied by two or more caregivers, the caregiver which shall interact with the monitor may be selected as the user.

In one embodiment, the recognition unit is configured to identify the subject and/or an object in the proximity of the user, the subject and/or the holder, and the control unit is configured to control the drive mechanism to automatically adjust the position and/or orientation of the holder based additionally on one or more of a position of the subject, a pose of the subject, a movement of the subject, a position of the object and a shape of the object. The position of the object may be a relative position between the position of the holder and the position of the object. The position of the subject may be a relative position between the position of the holder and the position of the subject.

The "proximity" may be related to the "usability" of the monitor to the primary caregiver, i.e., it may be related to the positioning of the monitor and the usage (i.e. interaction and/or visibility) of the monitor. The identification of the subject or an object can also be used to avoid collisions. In this sense "proximity" may be defined by the potential for collisions, which depends on the expected speed and range of motion (of the holder and eventually object and subject). The potential scenarios could be used to define the relevant "proximity" and to program the system accordingly. In practical situations, "proximity" may be in the range from several centimeters to several meters, e.g. between 10 cm and 5 m, depending on the particular scenario, transportation and application.

Therefore, collisions with the subject and/or the object may be avoided while the position and/or orientation of the holder is adjusted. The subject may be identified, e.g., by markers attached to the subject (e.g., pinned to clothing of the subject or plaster-like detachably glued to the skin), an optically readable tag, an NFC tag, an RFID tag, the position and/or pose of the subject in the room (e.g., due to lying on a stretcher), based on spatial-temporal information (e.g., the order in which persons entered the room and when which person is where in the room) and/or by exclusion procedure that this being is not the user. The object may be medical equipment which may be, e.g., handled by the user and/or a secondary caregiver.

Additionally or alternatively, the recognition unit may be configured to identify the secondary caregiver, wherein the control unit is configured to control the drive mechanism to automatically adjust the position and/or orientation of the holder based additionally on one or more of a position, pose and/or movement of the secondary caregiver. The secondary caregiver may provide medical care to the subject, e.g., by assisting the user. In contrast to the secondary caregiver, the user may exclusively or mainly operate the monitor and/or view the display of the monitor.

In an embodiment, the control unit is configured to determine whether the subject is in a critical state based on the subject information. For example, the control unit may use a predictive model and/or a threshold and/or determine trends and/or a predictive parameter related to the vital sign information to determine whether the subject is in a critical state.

In another embodiment, the control unit is configured to determine whether the subject is in a critical state based additionally on an eye tracking pattern of a user, in particular a caregiver of the subject, in particular an eye tracking pattern related to the monitor.

Therefore, in determining whether the subject is in a critical state, the control unit may take into account an assessment of the user regarding the current state of the subject. This is based thereon that the eye tracking pattern of the user may be different depending on the assessment of the user regarding the state of the subject. For example, the eye tracking pattern may indicate that the user is unsure whether the state of the subject deteriorates and/or is critical, e.g., because the user notices irregularities (such as new, changing and/or peculiar symptoms) and is worried. For example, the user may look at the display of the monitor (e.g., on a particular parameter of the vital signs) and/or the subject more frequently and/or for a longer time period than before. Depending on the eye tracking pattern, the control unit may control the drive mechanism to move the holder to the use state in order to provide the user with one or more parameter of the subject information and/or additional information based on the subject information such that the user may determine whether clinical intervention is necessary. Thus, the control unit may determine a critical state of the subject if the eye tracking pattern of the user indicates a critical state of the subject although, e.g., vital signs of the subject do not exceed a threshold. The eye tracking pattern of the user may be detected, e.g., by a recognition unit configured to detect eye movement of the user. The recognition unit may comprise a camera and software configured to analyze the eye tracking pattern of the user.

In a further embodiment, the control unit is configured to determine whether to move the holder from the parking state to the use state based additionally on information related to transportation of the subject, in particular information related to the physical movement of the subject during transportation, a geographical and/or temporal information on a route of transportation and/or deviations from the route of transportation. This may be applied to different transportation scenarios, including intra-hospital and inter-hospital transportation.

Information related to transportation of the subject may comprise, e.g., a start and/or target location of the transport (e.g., derived from the connection or disconnection of equipment and/or tracking), the route of transport (e.g., derived from tracking means), expected time for the transport, delay caused by a traffic jam and/or traffic lights, an alternative route taken, vibrations (e.g., due to different grounds on which the ambulance drives) and/or a change of the pose and/or position of the subject (e.g., when an ambulance drives up and/or down an incline and the subject no longer lies horizontal, or due to medical intervention), etc.. Vibrations acting on the subject may, e.g., be detected by a vibration detector and/or determined due to geographical and/or temporal information on the route of transportation. A change of pose of the subject may be detected, e.g., by sensors applied to the subject and/or by visual detection (e.g., a recognition unit configured to identify the position, pose and/or movement of the subject).

If the patient is near the end destination, actionability (e.g. if an intervention by the caregiver is possible or needed) and the decision if the holder should be moved into the use state may be related to the current location. If the patient has criticality that needs advanced intervention that is not available at the current location, a different response may be chosen than when the intervention is available at the current location. Information related to the transport of the patient may be used to decide whether information is actionable by the caregiver during transport (and therefore e.g. switch from parking state to use state, or not).

For instance, in case a critical situation of the patient is predicted in the near future, e.g. by a predictive algorithm or early warning score, it might be actionable for the user, when transporting the patient from the ICU to the imaging department (a possible action may be to postpone the imaging and return to the ICU). On the other hand, the same patient status might not be actionable when transporting the patient from the imaging department or the OR to the ICU, as it is anticipated that the patient will be monitored at the ICU, when the predicted deterioration of the patient status will happen (in this case, actionability might depend as well on how long it will take until the patient presumably arrives at the ICU, and therefore, information about (deviations from) the route of transportation might be useful), i.e., in the first case, it may be switched from parking state to use state, in the second case it would not.

On one embodiment, the holder is configured to be in a fail-safe state, wherein the control unit is configured to determine whether an error state is present based on an inconsistency in the subject information, input from a recognition unit configured to identify and/or track a user and/or information related to the transportation of the subject and/or a malfunction of the drive mechanism and/or the recognition unit, and the control unit is configured to control the drive mechanism to set the holder from the use state to the fail-safe state if an error state is determined.

The fail-safe state may be a state in which the position and/or orientation of the holder is relatively safe with regard to avoiding collisions (mechanical contact) with the user, the subject, a secondary caregiver and/or an object while the user can view the display of the monitor and operate the monitor, e.g., in case of alarms and/or other events. The fail-safe state may be a state in which the holder is static or certain degrees of freedoms of the holder are (temporarily) restricted. The fail-safe state may differ from the parking state and the use state. The fail-safe state may be a trade-off between the use state and the parking state. Compared to the parking state, the fail-safe state may be less safe in regard to, e.g., collisions with the user and/or the subject than the parking state but may allow for a more user-friendly operation of the monitor (in particular a better access to the monitor to operate the monitor). Compared to the use state, the fail-safe state may be safer, in particular in regard to collisions, but may allow for a less user-friendly operation of the monitor. For example, the holder is configured to be moved to the fail-safe state if a malfunction of the recognition unit is detected and the position and/or orientation of the holder should not be dynamically adjusted any more to avoid collisions with and damage to the monitor.

In an embodiment, the holder is configured to be in a manual state in which the holder is manually movable. In the manual state, the holder may be moved to a position and/or orientation desired by the user. For example, the manual state may be activatable by pressing a button. For example, the holder may be adjustable as long as the button is pressed. The button may be arranged at the holder in a way such that the user may press the button and adjust the position and/or orientation of the holder at the same time. The manual state may also be activatable by voice control, remote control and/or a touch screen (e.g., a touch screen of the monitor). Similarly, the position and/or orientation of the holder in the manual state may be controlled, e.g., manually (by mechanical force), by voice control, remote control and/or a touch screen.

In the manual state, degrees of freedom of the holder may be activatable in different stages. For example, the drive mechanism may comprise an arm mechanism with a plurality of joints configured to adjust the position and/or orientation of the holder. In the first stage, only some of the plurality of joints are moveable. In a second stage, further joints or the rest of the joints are movable. For example, the first stage may be activatable when pressing a button to the first position and the second stage may be activated when pressing the button to a second position. For example, pressing the button into the second position may require a greater force than pressing the button into the first position. Additionally or alternatively, tactile feedback may be provided so that the user may recognize whether the button is in the first position or the second position more easily.

When the manual state is activated, the control unit may be configured to control the drive mechanism to ensure basic safety. For example, the control unit may prevent the monitor from falling out of the receptacle of the holder. Additionally or alternatively, the control unit may prevent the holder and/or the monitor from colliding with the user, the subject and/or an object. Thus, damage and/or injury may be prevented in case the manual state is activated unintentionally by the user. When the manual state is deactivated, the control unit may automatically determine whether the holder should be subsequently moved to the parking state or the use state.

In a further embodiment, the device further comprises a projection unit configured to be in a sleep state and an alert state, wherein in the alert state, the projection unit is configured to project one or more parameters of subject information onto a target position, wherein the control unit is configured to control the projection unit to be set from the sleep state to the alert state if the subject information indicates that the subject is in acritical state.

This embodiment may be used in addition to other embodiments, but can also be itself an inventive improvement of prior art.

In the sleep state, the projection unit does not project any parameters of the subject information or may project fewer parameters of the subject information than in the alert state. In the sleep state, the projection unit may be in an energy-saving state. The control unit may set the projection unit to the alert state if the holder is in the use state. Therefore, the user may be provided with one or more parameters of the subject information which are relatively conveniently readable for the user additionally to information provided by the monitor in the use state.

Additionally or alternatively, the control unit may set the projection unit to the alert state if the holder is in the fail-safe state, the manual state and/or the use state, wherein in the use state, the holder cannot be adequately positioned and/or orientated (e.g., placed in an adequate manner to ensure safety with regard to collisions and at the same time ensuring a good view onto the display of the monitor). Therefore, the user may be provided with information on the state of the subject even, e.g., in case of a malfunction of the drive mechanism or if the device fails to provide an adequate interaction of the user with the monitor, in particular with regard to operation of the monitor and the view onto the display of the monitor.

Optionally, the device may comprise a recognition unit configured to identify a user, in particular a caregiver of the subject, wherein the control unit is configured to control the projection unit to automatically adjust the target position in the alert state based on position and/or pose and/or movement of the user. The target position may be controlled to be within the field of view of the user and/or a position that is easily visible to the user.

Furthermore optionally, the recognition unit may be configured to identify a position, pose and/or movement of the subject and/or a shape and/or position of an object in proximity of the user, wherein the control unit is configured to control the projection unit to automatically adjust the target position additionally on one or more of the position of the subject, pose of the subject, movement of the subject, shape of the object and position of the object.

Yet further optionally, when adjusting the target position, the recognition unit may take into account the background onto which the one or more parameters of the subject information are to be projected. For example, projection onto smooth surfaces may be preferred to projection onto an uneven surface, e.g., a surface with a plurality of grooves. Therefore, the legibility of the one or more parameters of the subject information may be enhanced. Furthermore, the information may be projected into/on glasses of the caregiver (also called "augmented reality"), e.g. as 2D projection or 3D holographic projection.

In another aspect, a device for monitoring a subject is provided, the device comprising:
- a projection unit configured to be in a sleep state and an alert state, wherein in the alert state, the projection unit is configured to project one or more parameters of subject information onto a target position; and
- a control unit configured to obtain subject information indicating whether the subject is in a critical state and to control the projection unit to be set from the sleep state to the alert state if the subject information indicates that the subject is in a critical state.

Therefore, the user may be provided with one or more parameters of the subject information such that the user may make a decision on clinical treatment.

In the sleep state, the projection unit does not project any parameters of the subject information or may project fewer parameters of the subject information than in the alert state. In the sleep state, the projection unit may be in an energy-saving state. In the alert state, the projection unit may project more parameters of the subject information than in the sleep state.

A critical state of the subject may be a state which requires attention and/or assessment of the state of the subject and/or clinical intervention by the user. This may in particular require providing the user with one or more parameters of the subject information on a display of the monitor. For example, the state of the subject may require that the user checks the state of the subject to determine whether clinical intervention is necessary. For example, the subject information may indicate that sensor information is lacking, e.g., because a sensor loosened and needs to be reattached or because additional measurement is advisable. The subject information may also indicate to the user that administration of medication is scheduled and/or that the state of the patient requires administration of (additional) medication. Furthermore, the critical state of the subject may indicate that the subject experiences a life-threatening situation. The user may be alerted to a critical state by an alarm, e.g., information displayed on the monitor or by an audible signal.

According to the aspect, the projection unit may be only in the alert state if necessary and/or only as long as necessary. In the case that no attention from the caregiver is necessary, the projection unit may be in the sleep state. As long as the projection unit is in the sleep state, the user may rely on the fact that the subject is likely in a non-critical condition. This may indicate to the user that less high attention is required such that the user does not have to be constantly at maximum concentration. This may allow the user to better manage time periods with very high concentration and time periods with less high concentration. Furthermore, less distraction of the user may be ensured such that the user may focus on the subject. Also, the risk of collisions with the projection unit and/or damage of the projection unit may be reduced, e.g., while the user provides routine clinical care during transport.

In another embodiment, the user may operate the monitor by interacting with a projected information, e.g. by means of a camera, which detects gestures of the user relatively to the projected information. Further, in an embodiment, the projection unit may project which gesture of the user would be recognized as which kind of command input. Then, there is no need for an additional display or even a monitor, or the monitor could always be in the parking position (as mentioned below, in one embodiment, as long as there is no malfunction of the projection unit detected).

In a further aspect, a system for monitoring a subject is provided, the system comprising:
- a monitor comprising a display for displaying the subject information; and
- a device for monitoring a subject as disclosed herein, wherein the device is configured to obtain the subject information from the monitor.

In yet another aspect, a method for monitoring a subject is provided, the method comprising:
- obtaining subject information indicating whether the subject is in a critical state;
- controlling the projection unit to be set from the sleep state to the alert state if the subject information indicates that the subject is in a critical state; and
- projecting one or more parameters of the subject information on a target plane if the projection unit is in the alert state.

In yet a further aspect, there is provided a corresponding computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

In one embodiment, the subject information comprises one or more of vital sign information of the subject, an alert, a trend and/or a predictive parameter related to vital sign information.

Based on a predictive algorithm and/or an early warning score, the projection unit may be in the sleep state during "safe time periods". The vital sign information may be obtained by a sensor deployed on the subject.

In an embodiment, the device further comprises a recognition unit configured to identify a caregiver of the subject, wherein the control unit is configured to control the projection unit to automatically adjust the target position in the alert state based on position and/or pose and/or movement of the caregiver.

Therefore, the user may move relatively freely without trying to maintain a certain position and/or pose in which the user can read the one or more parameters projected by the projection unit. Thus, it may be ensured that the user is permanently provided with the relevant information, when the projection unit is in alert state, even if the user changes position and/or pose and/or moves. For example, the projection unit may adjust the target position to maintain a set distance to the user, e.g., by moving the target position if the user, e.g., stretches out his arm in order to administer medication to the subject or if the user leans over the patient.

In a further embodiment, the recognition unit is configured to identify the caregiver by face recognition, eye movement tracking and/or real time locating, in particular a radio frequency identification, RFID, tag, a near field communication, NFC, tag and/or an optically readable information tag.

For example, face recognition software may be trained in advance to recognize the user, e.g., the caregiver who will take care of the patient during transport. Also, an eye gazing pattern may be used to distinguish the user from non-users. The recognition unit may be configured to compare an eye gazing pattern expected for a user familiar using the device and/or medical equipment with the eye gazing pattern of a potential user. This may allow to distinguish, e.g., the subject and/or a potential user (e.g., a secondary caregiver) from the user. For example, the user may be identified by an eye gazing pattern related to one or more parameters of the subject information displayed on the display of the monitor or an eye gazing pattern related to an alert and/or a notification on the display of the monitor. The optical readable information tag may be, for example an identity card of the user, a barcode, a QR-code and/or markers attached to the user (e.g., pinned to clothing of the user, plaster-like detachably glued to the skin and/or to a headgear). The RFID tag, the NFC tag and/or the optical readable information tag may be held to a defined position (e.g., near the monitor) to check in a desired user.

Alternatively or additionally, one of many possible users may be chosen on a display (e.g., the display of the monitor) which shows potential users, e.g., identified by all identity cards being in range. If the subject is accompanied by two or more caregivers, the main caregiver may be therefore selected as the user such that the target position may be adapted to the user as main caregiver.

In one embodiment, the recognition unit is configured to identify the subject and/or an object in the proximity of the user and/or the subject, and wherein the control unit is configured to control the projection unit to automatically adjust the target position based additionally on one or more of a position, a pose and/or a movement of the subject and/or a position and/or shape of the object. Therefore, the eligibility of the projected one or more parameters may be ensured.

In one embodiment, the control unit is configured to determine whether the subject is in a critical state based on the subject information.

For example, the control unit may use a predictive model and/or a threshold and/or determine trends and/or a predictive parameter related to the vital sign information to determine whether the subject is in a critical state.

In an embodiment, the control unit is configured to determine whether the subject is in a critical state based additionally on an eye tracking pattern of a caregiver of the subject, in particular an eye tracking pattern related to the monitor.

Therefore, in determining whether the subject is in a critical state, the control unit may take into account an assessment of the user regarding the current state of the subject. This is based thereon that the eye tracking pattern of the user may be different depending on the assessment of the user regarding the state of the subject. For example, the eye tracking pattern may indicate that the user is unsure whether the state of the subject deteriorates and/or is critical, e.g., because the user notices irregularities (such as new, changing and/or peculiar symptoms) and is worried. For example, the user may look at the display of the monitor (e.g., on a particular parameter of the vital signs) and/or the subject more frequently and/or for a longer time period than before.

In a further embodiment, the control unit is configured to determine whether the subject is in a critical state based additionally on information related to transportation of the subject, in particular information related to the physical movement of the subject during transportation, a geographical and/or temporal information on a route of transportation and/or deviations from the route of transportation.

Information related to transportation of the subject may comprise, e.g., a start and/or target location of the transport (e.g., derived from the connection or disconnection of equipment and/or tracking), the route of transport (e.g., derived from tracking means), expected time for the transport, delay caused by a traffic jam and/or traffic lights, an alternative route taken, vibrations (e.g., due to different grounds on which the ambulance drives) and/or a change of the pose and/or position of the subject (e.g., when an ambulance drives up and/or down an incline and the subject no longer lies horizontal, or due to medical intervention), etc.. Vibrations acting on the subject may, e.g., be detected by a vibration detector and/or determined due to geographical and/or temporal information on the route of transportation. A change of pose of the subject may be detected, e.g., by sensors applied to the subject and/or by visual detection (e.g., a recognition unit configured to identify the position, pose and/or movement of the subject).

In one embodiment, the device further comprises:
- a holder configured to hold a monitor, wherein the holder is configured to be in a parking state and a use state; and
- a driving unit configured to move the holder from the parking state to the use state; wherein the control unit is configured to move the holder from the parking state to the use state if a malfunction of the projection unit is detected. Therefore, providing the user with relevant information to make clinical decisions may be ensured even if a malfunction of the projection unit is present.

### BRIEF DESCRIPTION OF THE DRAWINGS

These and other aspects of the invention will be apparent from and elucidated with reference to the embodiment(s) described hereinafter. In the following drawings:
Fig. 1 shows a schematic diagram of an embodiment of a system according to the present invention.
Fig. 2 shows a schematic diagram of an embodiment of a device according to the present invention.
Fig. 3 shows a flow chart of an embodiment of a method according to the present invention.
Fig. 4 shows a flow chart of another embodiment of the method according to the present invention.
Fig. 5 shows a schematic diagram of another embodiment of the system according to the present invention.
Fig. 6 shows a schematic diagram of another embodiment of the device according to the present invention.
Fig. 7 shows a flow chart of another embodiment of the method according to the present invention.

### DETAILED DESCRIPTION OF EMBODIMENTS

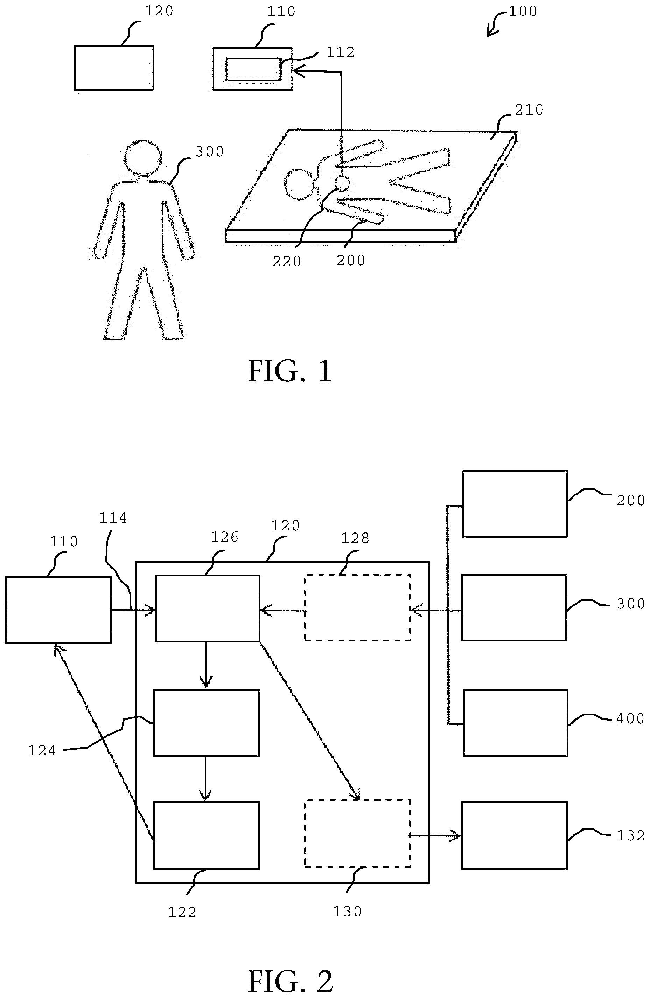

Fig. 1 shows a schematic diagram of an embodiment of a system 100 for automatically adjusting the position and/or orientation of a monitor 110, such as a display or a patient monitor, for monitoring a subject 200, according to the present invention. The subject 200 to be monitored lies on a stretcher 210. Sensor 220 is deployed on subject 200 for obtaining (i.e. retrieving or receiving) vital signs from the subject 200. A user 300 such as a caregiver is responsible for medical care of the subject 200 during transport. System 100 comprises a monitor 110 which is a patient monitor and comprises a display 112 and a device 120 for automatically adjusting the position and orientation of the monitor 110.

The monitor 110 obtains (i.e. retrieves or receives) the vital signs from sensor 220. The vital signs are displayed on display 112 (e.g., a touch screen) to provide the user 300 with information on the health status of the subject 200 such that the user 300 may make clinical decisions depending on the state of the subject 200.

Fig. 2 shows a schematic diagram of an embodiment of the device 120 for automatically adjusting the position and orientation of a monitor 110 according to the present invention. The device 120 comprises a holder 122 configured to hold the monitor 110, a driving mechanism 124 configured to move the holder 122 and a control unit 126 configured to control the driving mechanism 124.

The drive mechanism 124 may comprise an arm mechanism with a plurality of joints, wherein the holder 122 is fastened to the arm mechanism. The arm mechanism allows to move and orientate the holder 122 with six degrees of freedom. The holder 122 may be moved between a parking state and a use state by adjusting the joints of the arm mechanism. The use state of the holder 122 is configured for use of (interaction with) the monitor 110 by the user 300 such that the user 300 may operate the monitor 110 relatively conveniently and has a relatively good view onto the display 112 (see Fig. 1) while providing the subject 200 with medical care. The parking state is relatively safe in regard to collisions of the holder 122 and the monitor 110 with the user 300 and the subject 200. The monitor 110 may, e.g., be positioned close to an edge of the stretcher or in/behind the middle of the head end (other positions are generally possible as well).

Control unit 126 obtains subject information 114 from the monitor 110, wherein the subject information 114 comprises vital signs obtained from sensor 220. Based on the obtained subject information 114, the control unit 126 may determine whether the vital signs violate a threshold and/or whether a trend is present indicating that the state of the subject 200 deteriorates. Alternatively, determination whether the subject information 114 indicates that the subject is in a critical state may be performed by the monitor 110, wherein the control unit 126 may obtain an alert if the subject information 114 indicates that the subject is in a critical state. If the subject information 114 indicates that the subject 200 is in a critical state, the control unit 126 controls the drive mechanism 124 to move the holder 122 from the parking state to the use state such that the monitor 110 held by the holder 122 is moved as well.

Optionally, the device further comprises a recognition unit 128. The recognition unit 128 identifies the user 300 as medical personnel, e.g. by an optically readable identity tag worn by the user (or any other method as described above), and/or by tracking the eye movement of the user 300. The control unit may obtain information from the recognition unit 128 regarding the position, pose and/or movement of the user 300. Based on the information on position, pose and/or movement of the user 300, the control unit may control the drive mechanism 124 to automatically adjust the position and orientation of the holder 122 such that the risk of collisions of the holder 122 and the monitor 110 with the user 300 is reduced.

Additionally, the recognition unit 128 may identify the subject 200 based on the lying position of the subject 200 on stretcher 210. Also, the recognition unit 128 may identify an object 400 close to the user 300, the subject 200 and/or the holder 122.

Further optionally, the device 120 further comprises a projection unit 130 which may be in an alert state or a sleep state. In the alert state, the projection unit 130 projects one or more parameters of the subject information 114 on a target position 132 such as on the stretcher 210. In the sleep state, the projection unit 130 may be in an energy-saving mode. If the subject information 114 indicates that the subject 200 is in a critical state, the control unit controls the projection unit to be set to the alert state such that the projection unit projects one or more parameter of the subject information 114 on the target plane.

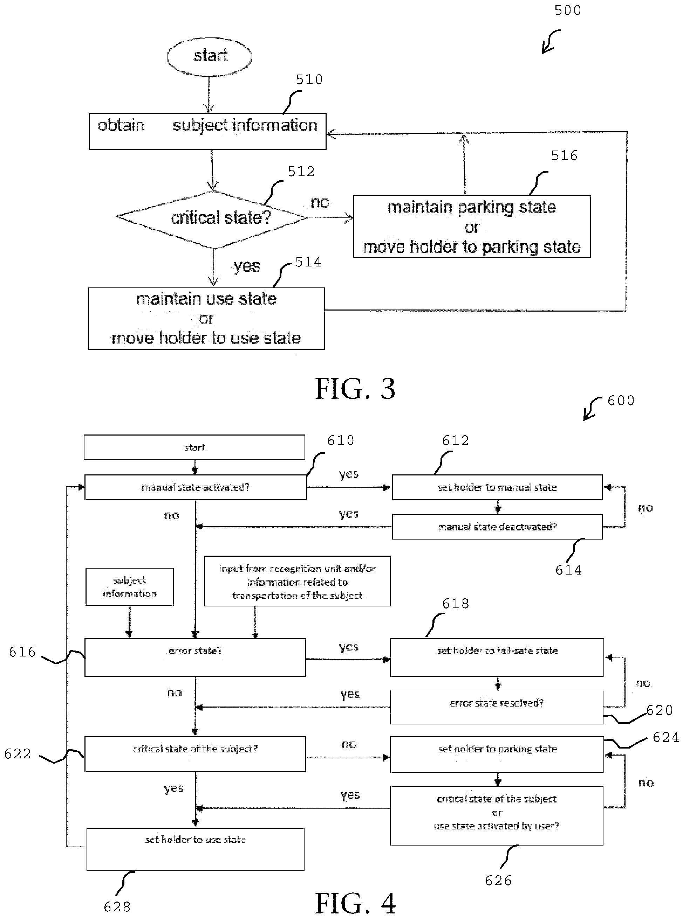

Fig. 3 shows a flow chart of a first embodiment of a method 500 for automatically adjusting the position and orientation of a monitor 110 according to the present invention. The steps of the method 500 may be carried out by the device 120, wherein the main steps of the method 500 are carried out by the control unit 126 and the drive mechanism 124. The method 500 may be implemented, e.g., as a computer program running on a computer or processor (e.g., on the processor of the monitor 110).

At the starting point, the holder 122 may be, e.g., in the parking state. In step 510, the control unit obtains (e.g., retrieves or receives) subject information 114 indicating whether the subject 200 is in a critical state. In step 512, the control unit 126 may determine whether the subject information 114 indicates that the subject 200 is in a critical state. Alternatively, the subject information 114 may already comprise the information that the subject 200 is in a critical state, e.g., in the form of an alert. In step 514, if the subject information 114 indicates that the subject 200 is in a critical state, the holder 122 is moved from the parking state to the use state. Otherwise, if the holder 122 is already in the use state, the use state is maintained. In step 516, if the subject information 114 indicates that the subject 200 is in a non-critical state, the holder 122 is moved from the use state to the parking state. Otherwise, if the holder 122 is already in the parking state, the parking state is maintained.

Fig. 4 shows a flow chart of another embodiment of the method 600 for automatically adjusting the position and orientation of a monitor 110 according to the present invention.

Compared to the embodiment of the method 500 shown in Fig. 3, the holder 122 may be additionally in a manual state and a fail-safe state. The manual state is a state in which the user 300 may manually adjust joints of the arm mechanism such that the position and orientation of the holder 122 can be adjusted. The user 300 may activate the manual state by pressing a button arranged on the holder 122 in proximity to the monitor 110. The fail-safe state is activated if an error state is present due to a malfunction (e.g., a malfunction of the arm assembly and/or the recognition unit 128) and/or an inconsistency in subject information 114, input from the recognition unit 128 and/or information related to the transportation of the subject 200. In the fail-safe state the holder 122 is statically positioned in a position allowing the user 300 to operate the monitor 110 while having an adequate view onto the display 112.

At the starting point, the holder 122 may be, e.g., in the parking state or use state. In step 610, the control unit 126 determines whether a manual state is activated by the user 300. In step 612, if the manual state is activated, the holder 122 is set to the manual state. In step 614, the control unit 126 determines whether the manual state has been deactivated. In step 616, if the manual state is not activated or deactivated, the control unit 126 determines whether an error state is present. The error state may be present due to an inconsistency in the subject information 114, input from the recognition unit 128 and/or information related to the transportation of the subject 200 and/or a malfunction of the drive mechanism 124 and/or the recognition unit 128. In step 618, if an error state is detected, the control unit 126 controls the drive mechanism 124 to move the holder 122 to the fail-safe state. In step 620, the control unit 126 determines whether the error state is resolved. In step 622, if no error state is detected or the error state is resolved, the control unit 126 determines whether the subject information 114 indicates that the subject 200 is in a critical state. Alternatively, the subject information 114 may already comprise the information that the subject 200 is in a critical state, e.g., in the form of an alert. In step 624, if the subject information 114 indicates that the subject 200 is in a non-critical state, the holder 122 is moved to the parking state (or maintained in the parking state). In step 626, starting from the parking state, the control unit 126 determines whether the use state is activated by the user 300 or the subject information 114 (in the meantime) indicates that the subject 200 is in a critical state. In step 628, if the use state is activated by the user 300 or the subject information 114 now indicates that the subject 200 is in a critical state, the holder 122 is moved to the use state (or maintained in the use state).

Additionally, the method 600 may include determining whether a switch off for the device 120 has been activated. Whether a switch off has been activated may be determined, e.g., in the use state, the parking state, the manual state and/or the fail-safe state. If the switch off has been activated, a switch off procedure of the device 120 is initiated. For example, the switch off procedure may comprise moving the holder 122 to the parking state.

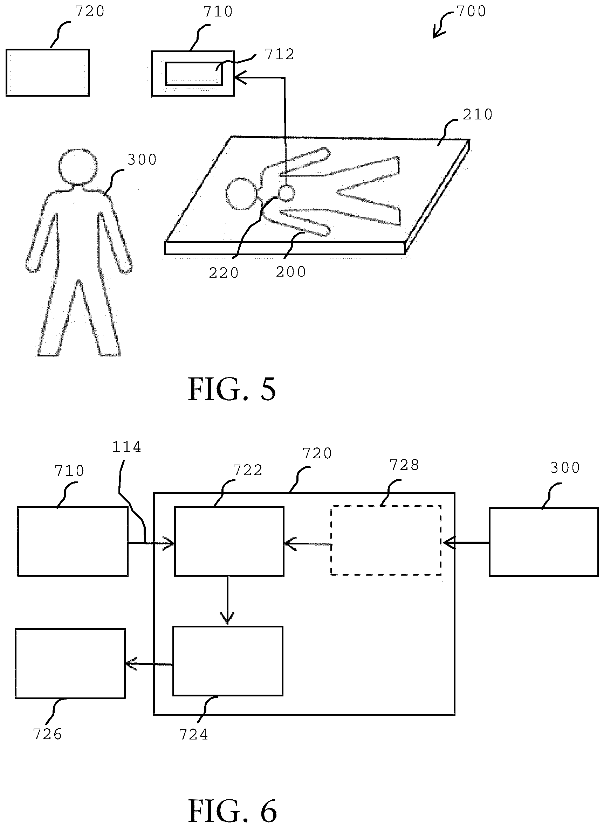

Fig. 5 shows a schematic diagram of another aspect of a system 700 for monitoring a subject 200 according to the present invention. The subject 200 to be monitored lies on a stretcher 210. A sensor 220 is deployed on the subject 200 for obtaining (i.e. retrieving or receiving) vital signs from the subject 200. A user 300 such as a caregiver is responsible for clinical care of the subject 200 during transport. System 700 comprises a monitor 710 (e.g., a patient monitor) with a display 712 (e.g., a touch screen) and a device 720 for monitoring the subject 200.

Fig. 6 shows a schematic diagram of an embodiment of the device 720 for monitoring a subject 200 according to the present invention. The device 720 comprises a control unit 722 and a projection unit 724. The projection unit 724 may be in an alert state or in a sleep state. In the alert state, the projection unit 724 projects one or more parameters of the subject information on a target position 726. In the sleep state, projection unit 724 is in an energy-saving mode.

The control unit 722 obtains (e.g., retrieves or receives) subject information 114 from the monitor 710, wherein the subject information 114 comprises the vital signs obtained from the sensor 220 and indicates whether the subject 200 is in a critical state or not. If the subject information 114 indicates that the subject 200 is in a critical state, the control unit 722 controls the projection unit 724 to be set to the alert state. If the subject information 114 indicates that the subject 200 is in a non-critical state, the control unit 722 controls the projection unit 724 to be set to the sleep state.

Optionally, the device 700 may comprise a recognition unit 728 which may identify the user 300. The recognition unit 728 may further detect a position, a pose and movements of the user 300. Based on the position, pose and movement of the user 300, the control unit 722 may control the projection unit 724 to automatically adjust the target position 726 to ensure that the user 300 may conveniently read the projection of the one or more parameters.

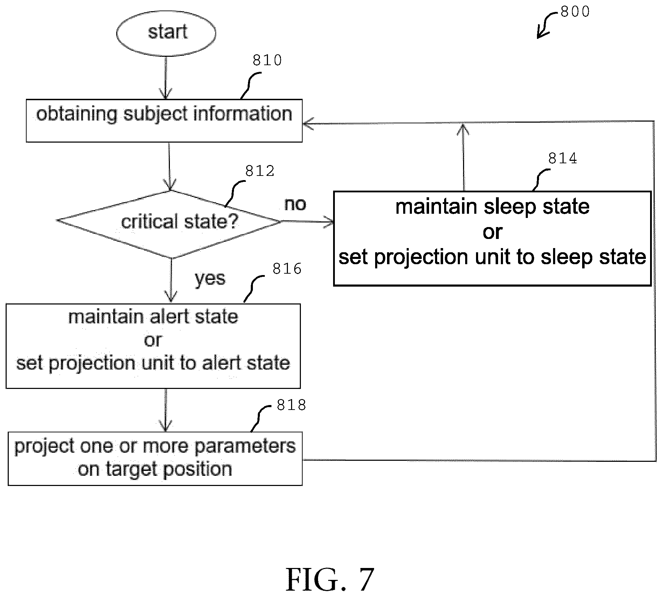

Fig. 7 shows a flow chart of a further embodiment of a method 800 for monitoring a subject 200 according to the present invention. At the starting point, the projection unit 724 may be, e.g., in the sleep state. In step 810, the control unit 722 obtains the subject information 114. In step 812, the control unit 722 determines whether the subject 200 is in a critical state based on the subject information 114. Alternatively, the subject information 114 may already comprise the information that the subject 200 is in a critical state, e.g., in the form of an alert. In step 814, if the subject is in a non-critical state, the projection unit 724 is set from the alert state to the sleep state or the sleep state is maintained if the projection unit 724 is already in the sleep state. In step 816, if the subject 200 is in a critical state, the projection unit 724 is set from the sleep state to the alert state or the alert state is maintained if the projection unit 724 is already in the alert state. Steps 810 to 818 may be repeated until, e.g., the device 720 is switched off.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (120) for automatically adjusting the position and/or orientation of a monitor, the device (120) comprising:
- a holder (122) configured to hold a monitor (110) configured to display subject information related to a subject's physiological condition, wherein the holder (122) is configured to be in a parking state and a use state;
- a drive mechanism (124) configured to move the holder (122) from the parking state to the use state; and
- a control unit (126) configured to obtain subject information (114) indicating whether the subject (200) is in a critical state and to control the drive mechanism to move the holder (122) from the parking state to the use state if the subject information (114) indicates that the subject (200) is in a critical state.

2. Device (120) according to claim 1,
wherein the subject information (114) comprises one or more of vital sign information of the subject (200), an alert, a trend and/or a predictive parameter related to vital sign information.

3. Device (120) according to any of the preceding claims,
wherein the control unit (126) is configured to control the drive mechanism (124) to automatically adjust position and/or an orientation of the holder (122) in the use state.

4. Device (120) according to any of the preceding claims,
further comprising a recognition unit (128) configured to identify a user (300), in particular a caregiver of the subject (200), wherein the control unit (126) is configured to control the drive mechanism (124) to automatically adjust position and/or an orientation of the holder (122) based on position and/or pose and/or movement of the user (300).

5. Device (120) according to claim 4,
wherein the recognition unit (128) is configured to identify the user (300) by face recognition, eye movement tracking and/or real time locating, in particular a radio frequency identification, RFID, tag, a near field communication, NFC, tag and/or an optically readable information tag.

6. Device (120) according to claim 4 or 5,
wherein the recognition unit (128) is configured to identify the subject (200) and/or is configured to identify an object (400) in the proximity of the user (300), the subject (200) and/or the holder (122), and
wherein the control unit (126) is configured to control the drive mechanism (124) to automatically adjust the position and/or orientation of the holder (122) based additionally on one or more of a position of the subject, a pose of the subject, a movement of the subject (200), a position of the object (400) and a shape of the object (400).

7. Device (120) according to any of the preceding claims,
wherein the control unit (126) is configured to determine whether the subject (200) is in a critical state based on the subject information (114).

8. Device (120) according to claim 7,
wherein the control unit (126) is configured to determine whether the subject (200) is in a critical state based additionally on an eye tracking pattern of a user (300), in particular a caregiver of the subject (200), in particular an eye tracking pattern related to the monitor (110).

9. Device (120) according to claim 7 or 8,
wherein the control unit (126) is configured to determine whether to move the holder (122) from the parking state to the use state based additionally on information related to transportation of the subject (200), in particular information related to the physical movement of the subject (200) during transportation, a geographical and/or temporal information on a route of transportation and/or deviations from the route of transportation.

10. Device (120) according to any of the preceding claims,
wherein the holder (122) is configured to be in a fail-safe state,
wherein the control unit (126) is configured to determine whether an error state is present based on an inconsistency in the subject information (114), input from a recognition unit (128) configured to identify and/or track a user (300) and/or information related to the transportation of the subject (200) and/or a malfunction of the drive mechanism (124) and/or the recognition unit (128) and
wherein the control unit (126) is configured to control the drive mechanism (124) to set the holder (122) from the use state to the fail-safe state if an error state is determined.

11. Device (120) according to any of the preceding claims,
wherein the holder (122) is configured to be in a manual state in which the holder (122) is manually movable.

12. Device (120) according to any of the preceding claims,
further comprising a projection unit configured to be in a sleep state and an alert state, wherein in the alert state, the projection unit (130) is configured to project one or more parameters of the subject information (114) onto a target position (132),
wherein the control unit (126) is configured to control the projection unit (130) to be set from the sleep state to the alert state if the subject information indicates that the subject (200) is in the critical state.

13. System (100) for automatically adjusting position and/or orientation of a monitor (110), the system (100) comprising:
- a device (120) according to any of the preceding claims; and
- the monitor (110), the monitor comprising a display (112) configured to display the subject information (114).

14. Method (500, 600) for automatically adjusting the position and/or orientation of a monitor (110), the method (500, 600) comprising:
- obtaining subject information (114) indicating whether the subject (200) is in a critical state;
- controlling a drive mechanism (124) to move a holder (122) configured to hold a monitor (110) configured to display subject information related to a subject's physiological condition from a parking state to a use state if the subject information (114) indicates that the subject (200) is in a critical state.

15. Computer program comprising program code means for causing a computer to carry out the steps of the method (500, 600) as claimed in claim 12 when said computer program is carried out on the computer.
